Europäisches Patentamt

European Patent Office

Office européen des brevets

(11) Veröffentlichungsnummer: **0 480 343 B1**

## EUROPÄISCHE PATENTSCHRIFT

(45) Veröffentlichungstag der Patentschrift: **13.12.95**

(21) Anmeldenummer: **91117013.2**

(22) Anmeldetag: **05.10.91**

(51) Int. Cl.⁶: **C12N 9/22**, //(C12N9/22, C12R1:465)

(54) **Typ II-Restriktionsendonuklease Ssp4800I**

(30) Priorität: **11.10.90 DE 4032213**

(43) Veröffentlichungstag der Anmeldung:
**15.04.92 Patentblatt 92/16**

(45) Bekanntmachung des Hinweises auf die Patenterteilung:
**13.12.95 Patentblatt 95/50**

(84) Benannte Vertragsstaaten:
**AT BE CH DE DK ES FR GB GR IT LI LU NL SE**

(56) Entgegenhaltungen:
**EP-A- 0 306 847**

**GENE, Bd. 92, Nr. 1/2, 16. August 1990, AMSTERDAM NL, Seiten 1 - 248; KESSLER, C. & MANTA, V. 'Specificity of restriction endonucleases and DNA modification methyltransferases - a review (edition 3)'**

(73) Patentinhaber: **BOEHRINGER MANNHEIM GMBH**

**D-68298 Mannheim (DE)**

(72) Erfinder: **Kaluza, Klaus, Dr.**
**Hochfeldanger 3**
**W-8173 Bad Heilbrunn (DE)**
Erfinder: **Frey, Bruno, Dr.**
**Hochfeldstrasse 50**
**W-8122 Penzberg (DE)**
Erfinder: **Jarsch, Michael, Dr.**
**Unterkarpfsee 11**
**W-8173 Bad Heilbrunn (DE)**

EP 0 480 343 B1

**Beschreibung**

Die Erfindung betrifft die neue Typ II-Restriktionsendonuklease Ssp4800I, ein verfahren zu ihrer Gewinnung und ihre Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolysiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht immer noch Bedarf an der Schaffung weiterer Typ II-Restriktionsendonukleasen, die für DNA-Sequenzen spezifisch sind und die bisher von keiner der bekannten Restriktionsendonukleasen erkannt werden. Der Erfindung liegt daher die Aufgabe zu Grunde, eine neue Restriktionsendonuklease zur Verfügung zu stellen, welche eine bisher von keinem derartigen Enzym erkannte Sequenz spezifisch zu erkennen und zu spalten vermag.

Gelöst wird diese Aufgabe erfindungsgemäß durch eine Typ-II-Restriktionsendonuklease mit der Erkennungssequenz und der durch die Markierung definierten Spaltstelle

$$\downarrow$$
$$\text{5'-T} \quad \text{GTAC} \quad \text{A-3'}$$
$$\text{3'-A} \quad \text{CATG} \quad \text{T-5'},$$
$$\uparrow$$

welche aus Mikroorganismen der Gattung Streptomyces erhältlich ist.

Gene, 92, Nr. 1/2, (1990), S. 1-248 beschreibt zwar die Erkennungssequenz TGTACA (S. 85, Tabelle 2). In diesem Dokument gibt es aber weder einen Hinweis auf die genaue Schnittstelle, noch auf einen Mikroorganismus, aus welchem das genannte Restriktionsenzym isoliert werden könnte.

Die erfindungsgemäße neue Restriktionsendonuklease, die im folgenden als Ssp4800I bezeichnet wird, hat ein Temperaturoptimum bei 50°C. Das Enzym besitzt eine gute Aktivität zwischen pH 7,5 und pH 8,5 in 10 mmol/l Tris/HCl-Puffer mit 1,0 mmol/l 2-Mercaptoethanol, 5 mmol/l $MgCl_2$ und 100 mmol/l NaCl. Das pH-Optimum liegt bei pH 8,0.

Die Erkennungssequenz läßt sich durch vollständigen Verdau der DNAs der Viren SV40 und Adeno 2, der Phagen Lambda und phiX174 und dem Phagenderivat M13mp7 sowie den Plasmiden pBR322 und pBR328 bestätigen. Diese DNA-Moleküle werden mit Ssp4800I behandelt.

Tabelle 1 zeigt einen Vergleich der experimentell beobachteten Schnittstellenspezifität mit einer computerermittelten Schnittstellenspezifität für ein Enzym, welches folgende Sequenz erkennt:

5'-TGTACA-3'

Tabelle I

| DNA | Anzahl der experimentell bestimmten Schnittstellen | Anzahl der durch Computeranalyse bestimmte Schnittstellen | Experimentell bestimmte Fragmentlängen (Basenpaare) | Durch Computeranalyse bestimmte Fragmentlängen (Basenpaare) | Spaltpositionen ermittelt durch Computeranalyse (bei Basenpaar) |
|---|---|---|---|---|---|
| SV40 | 2 | 2 | 3800 1400 | 3832 1411 | 3579 4990 |
| M13mp7 | 1 | 1 | 7200 | 7238 | 1025 |
| PhiX174 | 0 | 0 | 0 | 0 | 0 |
| pBR322 | 0 | 0 | 0 | 0 | 0 |
| pBR328 | 0 | 0 | 0 | 0 | 0 |
| Lambda | 5 | 5 | 16000 13500<br>9700 5200<br>3100 922 | 16002 13537<br>9713 5224<br>3104 922 | 5224 6146 15859<br>29396 32500 |
| Ad2 | 5 | 5 | 12000 6800<br>6700 5900<br>2700 2300 | 11614 6811<br>6682 5873<br>2710 2247 | 2247 8120 19734<br>22444 29126 |

Die Spaltposition innerhalb der Erkennungssequenz des Enzyms läßt sich an einem M13-Derivat, das im Abstand von ca. 30 - 200 Basen zur Bindungsstelle des universalen Sequenzierprimers (Messing, J. et al. (1981) Nucl. Acids Res. 9, 309 - 321) diese Erkennungssequenz trägt, bestimmen. An einzelsträngiger DNA des M13-Derivates werden zunächst mit dem universalen Sequenzierprimer Sequenzreaktionen nach der Didesoxy-Kettenabbruchmethode durchgeführt (Sanger, F., et al. (1977) Proc. Natl. Acad. Sci. USA 74, 560 - 564, Messing, J. et al. (1981) Nucl. Acids Res. 9, 309 - 321).

Parallel dazu wird der Sequenzierprimer mit T4-Polynukleotid-Kinase und [$^{32}$P]ATP am 5'-Ende radioaktiv markiert. Nach Anhybridisieren dieses 5'-endmarkierten Sequenzierprimers an die einzelsträngige M13-DNA wird in einer Auffüllreaktion mit DNA Poly-merase I, (Klenow Enzym) und einer Desoxynukleotidtriphosphatmischung aus dATP, dCTP, dGTP und dTTP eine partiell doppelsträngige DNA hergestellt. Diese DNA, deren neusynthetisierter Strang am 5'-Ende radioaktiv markiert ist, wird mit der Restriktionsendonuklease Ssp4800I gespalten. Die Hälfte des Spaltansatzes wird zusätzlich noch mit T4 DNA Polymerase in Gegenwart einer Mischung aller vier Desoxynukleotidtriphosphate behandelt, um glatte DNA-Enden zu erhalten.

Die Analyse der Reaktionsprodukte erfolgt durch Elektrophorese auf Sequenziergelen (8 mol/l Harnstoff, 5 % Polyacrylamid) und anschließender Autoradiographie. Die Interpretation der Ergebnisse wird nach Brown, N.L. und Smith, M. (Methods in Enzymology 65 (1980) 391 - 401) durchgeführt. Durch Vergleich der Laufstrecken der radioaktiv markierten Fragmente mit der Sequenzleiter wird die Lage der Spaltstelle bestimmt. Die zusätzlich mit T4 DNA Polymerase behandelten Proben zeigen im Vergleich zu der lediglich mit Ssp4800I gespaltenen Probe eine um 4bp verlängerte Laufstrecke der Banden. Damit ist gezeigt, daß Ssp4800I ein um 4bp 5'-überhängendes DNS-Ende erzeugt. Die Spaltung von Ssp4800I erfolgt innerhalb der Erkennungs-sequenz daher mit folgender Spezifität:

$$5'-T\,|\,GTAC\ A-3'$$
$$3'-A\ CATG\,|\,T-5'$$

Die experimentell bestimmte Anzahl der Schnittstellen ist identisch mit der Anzahl der Schnittstellen, die durch Computeranalyse mit den verschiedenen DNAs für die Sequenz
5'-TGTACA-3'
erhalten wurde (Tabelle I). Zusätzlich wurden diese Daten noch mit den Tabellen aus Gene 10 (1980) 357 - 370, verglichen.

Vorzugsweise wird Ssp4800I gewonnen, indem man Mikroorganismen der Gattung Streptomyces züchtet und das Enzym aus den Zellen gewinnt. Besonders bevorzugt wird der Stamm Steptomyces spec. BMTU 4800 (DSM 6181) verwendet.
Der Mikroorganismus Spreptomyces spec. BMTU 4800 ist bei der Deutschen Sammlung von Mikroorganismen (DSM), Mascheroder Weg 16, 3300 Braunschweig, BRD, hinterlegt und trägt die Hinterlegungsnummer DSM 6181.

Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Lambda-DNA. Die erhaltenen DNA-Fragmente werden in Agarosegelen in den für die Fragmentauftrennung üblichen Puffersystemen, in Gegenwart von Ethidiumbromid, elektrophoretisch aufgetrennt.

Die für die Gewinnung des Enzyms verwendeten Mikroorganismen wachsen aerob in M111-Medium (10 g/l yeast extract, 10 g/l Malzextrakt).
Die optimalen Wachstumsbedingungen sind bei 26 ° C, pH 6,5 - 7,5. Die Verdoppelungszeit beträgt etwa 2,5 Stunden.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen über eine French-Presse aufgeschlossen. Die weitere Reinigung des Überstands wird vorzugsweise über Affinitätschromatographie, und Ionentauscherchromatographie durchgeführt. Als Material für die Affinitätschromatographie geeignet ist beispielsweise Heparin-Sepharose CL-6B (Pharmacia). Ein geeigneter Kationenaustauscher ist beispielsweise Phospho-Cellulose (Whatman).

Als Anionentauscher geeignet ist das unter dem Namen DEAE-Sephacel (Pharmacia) erhältliche Produkt. Aber auch andere Chromatographiematerialien, die dem Fachmann bekannt sind, sind geeignet.

Die folgenden Beispiele erläutern die Erfindung weiter.

Beispiel 1

Der Mikroorganismus Streptomyces spec. BMTU 4800 DSM 6181 wird bei 26°C 10-12 Stunden gezüchtet und am Ende der logarithmischen Phase geerntet. Als Kulturmedium wird M111-Medium verwendet.

Die Zellpaste (30 g Naßgewicht) wird in 2,4 Volumen Puffer A (40 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch$^2$ aufgeschlossen und der Niederschlag abgetrennt. Der Überstand wird mit NH$_4$Cl versetzt (Endkonzentration 0,3 mol/l). Durch eine Polymin-Fällung werden die Nucleinsäuren entfernt. Anschließend wird der abzentrifugierte Überstand mit 60% (w/v) Ammoniumsulfat behandelt und der Niederschlag über eine Heparin-Sepharose-Säule fraktioniert. Zur Elution wird ein Gradient von 0 - 1 mol/l NaCl verwendet. Ssp4800I wird in den Fraktionen zwischen 0,5 und 0,7 mol/l NaCl gefunden. Die aktiven Fraktionen werden gegen Puffer B (40 mmol/l Tris-HCl, pH 8,0; 0,1 mmol/l EDTA; 7 mmol/l 2-Mercaptoethanol; 10% (v/v) Glycerin) equilibriert und auf einer DEAE-fast-flow-Säule fraktioniert. Zur Elution wird ein Gradient von 0 - 0,5 mol/l NaCl verwendet. Die aktiven Fraktionen werden gegen Puffer B dialysiert.

Anschließend werden sie auf eine Phospho-Cellulose-Säule, welche mit Puffer B equilibriert wurde, aufgegeben. Zur Elution wird ein Gradient von 0 - 1 mol/l NaCl in Puffer B verwendet.

Die aktiven Fraktionen werden Zusammengegeben und gegen Lagerpuffer (20 mmol/l Tris-HCl, pH 8,0, 10 mmol/l 2-Mercaptoethanol, 100 mmol/l NaCl, 0,1 mmol/l EDTA und 50% (v/v) Glycerin) dialysiert.

Beispiel 2

Bestimmung der Aktivität

Definition der Enzymeinheiten: 1 U Ssp4800I spaltet 1 μg Lamda-DNA innerhalb 1 Stunde bei 50°C in 25 μl Endvolumen.

Zu einer Mischung von 2,5 μl Inkubationspuffer (100 mmol/l Tris-HCl, pH 8,0, 50°C, 50 mmol/l Magnesiumchlorid, 1 mol/l NaCl und 10 mmol/l 2-Mercaptoethanol) werden 17,9 μl Wasser und 3,6 μl Lambda DNA (optische Dichte: 5,6 OD/ml) sowie 1 μl Ssp4800I-Lösung (1 U/μl) zugegeben. Die Lösung wird eine Stunde bei 37°C inkubiert, auf Eis gekühlt und mit 5 μl eines Abstopp-Reagenses, bestehend aus 7 mmol/l Harnstoff, 20 % (w/v) Saccharose, 60 mmol/l EDTA und 0,01 % (w/v) Bromphenolblau versetzt. Anschließend wird eine Trennung durch Elektrophorese in 1 % Agarose-Gelen für 3 - 4 Stunden bei 100 V durchgeführt. Die erhaltenen Banden werden über Vergleich mit einem DNA-Längen-standard identifiziert.

**Patentansprüche**

1.  Typ II-Restriktionsendonuklease mit der Erkennungssequenz und der durch die Markierung charakterisierten Schnittstelle

$$\downarrow$$
$$5'\text{-T} \quad \text{GTAC} \quad \text{A-}3'$$
$$3'\text{-A} \quad \text{CATG} \quad \text{T-}5'$$
$$\uparrow$$

erhältlich aus Mikroorganismen der Gattung Streptomyces.

2.  Restriktionsendonuklease nach Anspruch 1 dadurch gekennzeichnet, daß sie aus Streptomyces spec. BMTU 4800 (DSM 6181) erhältlich ist.

3.  Restriktionsendonuklease nach den Ansprüchen 1 oder 2, gekennzeichnet durch ein Temperatur-Optimum bei 50°C und ein pH-Optimum bei 8,0.

4.  Verfahren zur Gewinnung einer Typ II-Restriktionsendonuklease mit der Erkennungssequenz und der durch die Markierung charakterisierten Spaltstelle

↓

5'-T  GTAC  A-3'

3'-A  CATG  T-5'

↑

dadurch gekennzeichnet, daß man Mikroorganismen der Gattung Streptomyces züchtet und das Enzym aus den Zellen gewinnt.

5. Verfahren nach Anspruch 4, dadurch gekennzeichnet, daß man Streptomyces spec. BMTU 4800 (DSM 6181) züchtet.

6. Verfahren nach den Ansprüchen 4 und 5, dadurch gekennzeichnet, daß man die Zellen aufschließt und den Zellüberstand gewinnt.

7. Verfahren nach Anspruch 6, dadurch gekennzeichnet, daß man zur Feinreinigung den Zellüberstand einer Affinitätschromatographie, einer Anionenaustauschchromatographie und einer Kationenaustauschchromatographie unterwirft.

8. Verfahren nach Anspruch 7, dadurch gekennzeichnet, daß man zur Affinitätschromatographie trägerfixiertes Heparin verwendet.

9. Verwendung einer Typ-II-Restriktionsendonuklease mit der Erkennungssequenz und der durch die Markierung charakterisierten Schnittstelle

↓

5'-T  GTAC  A-3'

3'-A  CATG  T-5'

↑

erhältlich aus Mikroorganismen der Gattung Streptomyces zur Erkennung und Spaltung der kompletären doppel-strängigen DNA-Sequenz
     5'-TGTACA-3'

**Claims**

1. Type II restriction endonuclease with the recognition sequence and the cleavage position characterised by the marking

5'-T↓GTAC A-3'
3'-A CATG↑T-5'

obtainable from micro-organisms of the genus Streptomyces.

2. Restriction endonuclease according to claim 1, characterised in that it is obtainable from Streptomyces spec. BMTU 4800 (DSM 6181).

3. Restriction endonuclease according to claims 1 or 2, characterised by a temperature optimum at 50°C and a pH optimum at 8.0.

EP 0 480 343 B1

4. Process for the obtaining of a type II restriction endonuclease with the recognition sequence and the cleavage position characterised by the marking

$$5'-T{\downarrow}GTAC\ A-3'$$
$$3'-A\ CATG{\uparrow}T-5'$$

characterised in that one cultures micro-organisms of the genus Streptomyces and obtains the enzyme from the cells.

5. Process according to claim 4, characterised in that one cultures Streptomyces spec. BMTU 4800 (DSM 6181).

6. Process according to claims 4 and 5, characterised in that one digests the cells and obtains the cell supernatant.

7. Process according to claim 6, characterised in that, for the fine purification, one subjects the cell supernatant to an affinity chromatography, to an anion exchange chromatography and to a cation exchange chromatography.

8. Process according to claim 7, characterised in that one uses carrier-fixed heparin for the affinity chromatography.

9. Use of a type II restriction endonuclease with the recognition sequence and the cleavage position characterised by the marking

$$5'-T{\downarrow}GTAC\ A-3'$$
$$3'-A\ CATG{\uparrow}T-5'$$

obtainable from micro-organisms of the genus Streptomyces for the recognition and cleavage of the complementary double-stranded DNA sequence
5'-TGTACA-3'.

**Revendications**

1. Endonucléase de restriction de type II, avec la séquence de reconnaissance et le site de coupure caractérisé par le marqueur

$$\downarrow$$
$$5'-T\ GTAC\ A-3'$$
$$3'-A\ CATG\ T-5'$$
$$\uparrow$$

qui peut être obtenue à partir de micro-organismes du genre Streptomyces.

2. Endonucléase de restriction selon la revendication 1, caractérisée en ce qu'elle peut être obtenue à partir de Streptomyces spec. BMTU 4800 (DSM 6181).

3. Endonucléase de restriction selon la revendication 1 ou 2, caractérisée par une température optimale de 50°C et un pH optimal de 8,0.

7

4. Procédé de préparation d'une endonucléase de restriction de type II, avec la séquence de reconnaissance et le site de coupure caractérisé par le marqueur

$$\downarrow$$
$$5'\text{-}T \quad GTAC \quad A\text{-}3'$$
$$3'\text{-}A \quad CATG \quad T\text{-}5'$$
$$\uparrow$$

caractérisé en ce que l'on cultive des micro-organismes du genre Streptomyces et l'on récupère l'enzyme à partir des cellules.

5. Procédé selon la revendication 4, caractérisé en ce que l'on cultive Streptomyces spec. BMTU 4800 (DSM 6181).

6. Procédé selon la revendication 4 ou 5, caractérisé en ce que l'on effectue la rupture des cellules et l'on récupère le surnageant cellulaire.

7. Procédé selon la revendication 6, caractérisé en ce que pour la purification fine du surnageant cellulaire, on soumet celui-ci à une chromatographie d'affinité, une chromatographie par échange d'ions et une chromatographie d'échange cationique.

8. Procédé selon la revendication 7, caractérisé en ce que pour la chromatographie d'affinité, on utilise de l'héparine fixée à un support.

9. Utilisation d'une endonucléase de restriction de type II, avec la séquence de reconnaissance et le site de coupure caractérisé par le marqueur

$$\downarrow$$
$$5'\text{-}T \quad GTAC \quad A\text{-}3'$$
$$3'\text{-}A \quad CATG \quad T\text{-}5'$$
$$\uparrow$$

qui peut être obtenue à partir de micro-organismes du genre Streptomyces, pour la reconnaissance et la coupure de la séquence d'ADN double brin complémentaire
   5'-TGTACA-3'.